# EUROPEAN PATENT SPECIFICATION

(11) **EP 1 905 477 B1**
(45) Date of publication and mention of the grant of the patent: **19.08.2009**
(21) Application number: 07023181.6
(22) Date of filing: 29.03.2004
(51) Int. Cl.: A61M 31/00, A61F 2/82, A61K 9/00

(54) **Beneficial agent delivery system**
System zur Freisetzung eines Heilmittels
Système d'administration d'agent utile

(30) Priority: 28.03.2003 US 458906 P; 26.03.2004 US 810241
(43) Date of publication of application: 02.04.2008
(62) Divisional of application: 04758552.6
(73) Proprietor: Conor Medsystems, Inc., Menlo Park, CA 94025 (US)
(72) Inventor: Shanley, John F., Redwood City, CA 94061 (US); Parker, Theodore J., Danville, CA 94506 (US); Eigler, Neal L., Pacific Palisades, CA 90272 (US)
(74) Representative: Schorr, Frank Jürgen

(56) References cited:
- EP-A- 1 277 449
- WO-A-03/015664
- US-A- 4 989 601
- US-A1- 2002 082 679
- US-A1- 2002 128 704

## Description

### Field of the Invention

The invention relates to an implantable medical device and method for delivery of an agent to a location within a patient, and more particularly, the invention relates to the selective modulation of agent delivery from the implantable medical device.

### Description of the Related Art

Implantable medical devices are often used for delivery of a beneficial agent, such as a drug, to an organ or tissue in the body at a controlled delivery rate over an extended period of time. These devices may deliver agents to a wide variety of bodily systems to provide a wide variety of treatments.

One of the many implantable medical devices which have been used for local delivery of beneficial agents is the vascular stent. Vascular stents are typically introduced percutaneously, and transported transluminally until positioned at a desired location. These devices are then expanded either mechanically, such as by the expansion of a mandrel or balloon positioned inside the device, or expand themselves by releasing stored energy upon actuation within the body. Once expanded within the lumen, these devices, called stents, become encapsulated within the body tissue and remain a permanent implant.

Known stent designs include monofilament wire coil stents (U.S. Pat. No. 4,969,458); welded metal cages (U.S. Pat. Nos. 4,733,665 and 4,776,337); and, most prominently, thin-walled metal cylinders with axial slots formed around the circumference (U.S. Pat. Nos. 4,733,665; 4,739,762; and 4,776,337). Known construction materials for use in stents include polymers, organic fabrics and biocompatible metals, such as stainless steel, gold, silver, tantalum, titanium, cobalt based alloys, and shape memory alloys, such as Nitinol.

Of the many problems that may be addressed through stent-based local delivery of beneficial agents, one of the most important is restenosis. Restenosis is a major complication that can arise following vascular interventions such as angioplasty and the implantation of stents. Simply defined, restenosis is a wound healing process that reduces the vessel lumen diameter by extracellular matrix deposition, neointimal hyperplasia, and vascular smooth muscle cell proliferation, and which may ultimately result in renarrowing or even reocclusion of the lumen. Despite the introduction of improved surgical techniques, devices, and pharmaceutical agents, the overall restenosis rate is still reported in the range of 25% to 50% within six to twelve months after an angioplasty procedure. To treat this condition, additional revascularization procedures are frequently required, thereby increasing trauma and risk to the patient.

One of the techniques under development to address the problem of restenosis is the use of various beneficial agents in or on stents. U.S. Pat. No. 5,716,981, for example, discloses a stent that is surface-coated with a composition comprising a polymer carrier and paclitaxel (a well-known compound that is commonly used in the treatment of cancerous tumors). The patent offers detailed descriptions of methods for coating stent surfaces, such as spraying and dipping, as well as the desired character of the coating itself: it should "coat the stent smoothly and evenly" and "provide a uniform, predictable, prolonged release of the anti-angiogenic factor." Surface coatings, however, can provide little actual control over the release kinetics of beneficial agents. These coatings are necessarily very thin, typically 5 to 8 microns deep. The surface area of the stent, by comparison is very large, so that the entire volume of the beneficial agent has a very short diffusion path to discharge into the surrounding tissue.

Increasing the thickness of the surface coating has the beneficial effects of improving drug release kinetics including the ability to control drug release and to allow increased drug loading. However, the increased coating thickness results in increased overall thickness of the stent wall. This is undesirable for a number of reasons, including increased trauma to the vessel wall during implantation, reduced flow cross-section of the lumen after implantation, and increased vulnerability of the coating to mechanical failure or damage during expansion and implantation. Coating thickness is one of several factors that affect the release kinetics of the beneficial agent, and limitations on thickness thereby limit the range of release rates, duration of drug delivery, and the like that can be achieved.

In addition to sub-optimal release profiles, there are further problems with surface coated stents. The fixed matrix polymer carriers frequently used in the device coatings typically retain approximately 30% - 80% of the beneficial agent in the coating indefinitely. Since these beneficial agents are frequently highly cytotoxic, sub-acute and chronic problems such as chronic inflammation, late thrombosis, and late or incomplete healing of the vessel wall may occur. Additionally, the carrier polymers themselves are often highly inflammatory to the tissue of the vessel wall. On the other hand, use of biodegradable polymer carriers on stent surfaces can result in the creation of "virtual spaces" or voids between the stent and tissue of the vessel wall after the polymer carrier has degraded, which permits differential motion between the stent and adjacent tissue. Resulting problems include micro-abrasion and inflammation, stent drift, and failure to re-endothelialize the vessel wall.

One drawback of known stents with drugs in or on the stents is the inability to tailor drug delivery to the tissue structure present adjacent the implanted device. The drug delivered from a drug delivery stent is delivered to all the tissue supported by the stent including both the diseased tissue (lesion) and neighboring relatively healthy tissue. The delivery of an anti-restenosis drug or other drugs to relatively healthy tissue in addition to the tissue to be treated may in some cases cause damage to this relatively healthy tissue. For example, aneurysms have been observed to form in the wall of a blood vessel in a portion of the blood vessel which is supported by the stent and has no apparent lesions, i.e. the relatively healthy tissue close to the diseased tissue, while similar aneurysms have not been observed in the diseased tissue. However, due to a combination of stent placement inaccuracies and a requirement to support the entire diseased tissue site, a stent must support both the diseased tissue and some relatively healthy tissue. It may also be desirable to deliver variable amounts of the drug to different tissue for many other reasons.

Patent application US 2002/0128704 A1 discloses a method for controlling the activity of drugs on or in drug-coated or drug-loaded implantable metallic devices. The method uses inductive heating of such devices. The heating of the device is used to activate drugs that have little activity at body temperature, to enhance for defined periods the reactive environment at the stent for drug-adjacent tissue interactions, or to deactivate drugs upon heating, employing reverse effects.

Accordingly, it would be desirable to provide an implantable medical device for delivery of agents, such as drugs, to a patient and selectively modulating, activating, or deactivating agent delivery after implantation to provide agents at targeted tissue areas adjacent the expandable medical device.

### Summary of the Invention

The present invention provides a system according to claim 1.

An implantable medical device may include a device body containing a beneficial agent arranged for delivery from the device body to an implantation site within a patient. The beneficial agent is configured to be selectively modulated after implantation within the patient by an activating/deactivating means. Furthermore, the beneficial agent at a first region of the device body can be modulated by the activating/deactivating means to create a different agent delivery profile than the beneficial agent at a second region of the device body.

The present invention provides an implantable medical device and a selective modulation catheter. The implantable medical device includes a beneficial agent arranged for delivery to an implantation site within a patient, where the beneficial agent is configured to be modulated after implantation within the patient by an activating/deactivating means. The selective modulation catheter has an activating/deactivating means configured to activate or deactivate the beneficial agent on a first region of the medical device to create a different delivery profile than the beneficial agent on a second region of the medical device.

In another beneficial agent delivery system aspect, the present invention provides an expandable implantable stent, a beneficial agent affixed to the stent, and an activating/deactivating means. The beneficial agent has an initial agent release profile, which can be modulated by the activating/deactivating means after implantation of the stent within a patient. This creates an agent release profile different from the initial agent release profile.

In a method aspect, the present invention may be used in a method of beneficial agent delivery with selective modulation of beneficial agent delivery. The method involves at least the following steps: 1) implanting an implantable medical device within a patient, where the device includes a beneficial agent; 2) delivering an activation means to a location of the implanted medical device; and, 3) modulating the amount of drug delivered from a first region of the implanted medical device with the activation/deactivation means without modulating the amount of beneficial agent delivered from a second region of the implanted medical device.

In another method aspect, the present invention may be used in a method of beneficial agent delivery from a stent. The method involves at least the following steps: 1) implanting a stent that includes a first beneficial agent and a second benefical agent within a lumen; 2) delivering the first beneficial agent from the stent; 3) determining whether the second beneficial agent is to be delivered; 4) delivering an activation means to the stent; and, 5) modulating the amount of second beneficial agent delivered from the stent with the activation means.

In a further method aspect, the present invention may be used in a method of beneficial agent delivery from a stent. The method involves at least the following steps: 1) implanting a stent that includes a first beneficial agent; 2) delivering the first beneficial gent from the stent; 3) determining when delivery of the first beneficial agent is to be terminated; 4) delivering a deactivation means to the stent; and, 5) substantially terminating the amount of second beneficial agent delivered from the stent with the deactivation means.

### Brief Description of the Drawing Figures

The invention will now be described in greater detail with reference to the preferred embodiments illustrated in the accompanying drawings, in which like elements bear like reference numerals, and wherein:

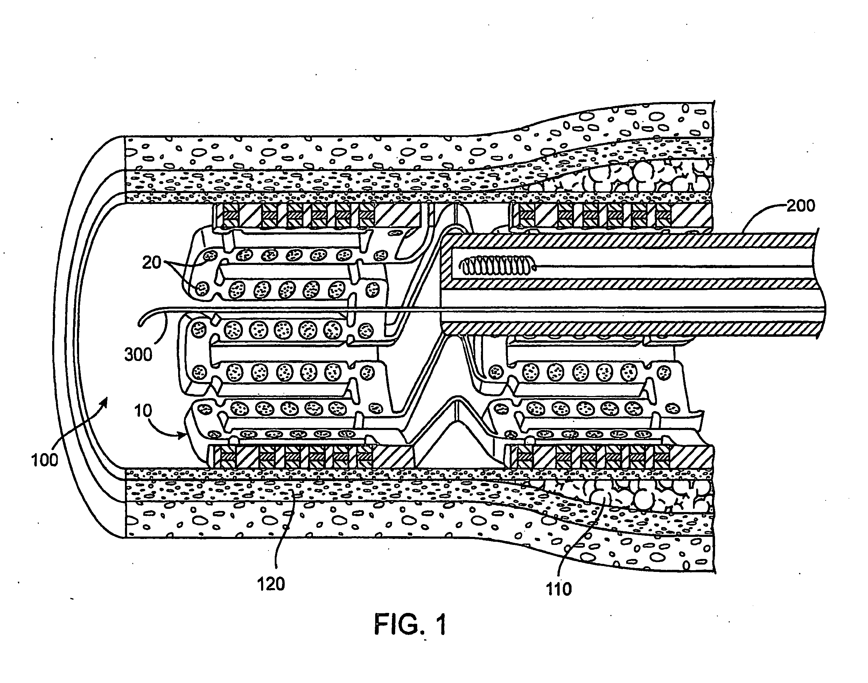

FIG. 1 is a side cross sectional view of an implantable beneficial agent delivery device and activation/deactivation means according to one example of the invention.

### Detailed Description of the Invention

The device of the invention comprises a stent and a drug delivery material therein or thereon such that the amount of drug delivered from one region of the stent can be modulated relative to the amount delivered from another region of the stent, such differential modulation being specified after the deployment of the stent into a mammal.

FIG. 1 illustrates an implantable medical device 10 implanted within a blood vessel 100. As shown in FIG. 1, the blood vessel 100 includes a lesion 110 which has been expanded by expansion of the medical device 10. In addition to supporting the lesion 110 or other unhealthy tissue, the expanded medical device 10 also supports one or more regions of relatively healthy blood vessel tissue 120. In the illustrated example, the relatively healthy tissue 120 is adjacent an end region of the medical device 10.

The expandable medical device 10 includes a plurality of openings 20 containing a beneficial agent to be delivered to the tissue. Some of the openings 20 are adjacent the lesion 110 and some of the openings are adjacent to the relatively healthy tissue 120. The present invention provides an activating/deactivating device in the form of a catheter 200 which is configured to be delivered over a guidewire 300 into the lumen of the medical device 10 after implantation to activate and/or deactivate the beneficial agent(s) within the openings 20 to provide targeted modulation of delivery of the beneficial agent to deliver the beneficial agent to specific regions or locations from a surface of the implanted medical device 10.

In the case of a beneficial agent which is useful for reducing extracellular matrix deposition, neointimal hyperplasia, and vascular smooth muscle cell proliferation, which may ultimately result in renarrowing or even reocclusion of the lumen the beneficial agent can be activated adjacent the lesion 110 or deactivated adjacent to relatively healthy tissue 120 to deliver the majority of the beneficial agent, preferably a therapeutically effective amount of the beneficial agent, to the targeted tissue.

The term "modulation" refers to the adjustment of the amount of beneficial agent delivered or the beneficial agent delivery rate either by activating or deactivating. Modulation of the amount of drug delivered is envisioned to include the range from no delivery of drug contained in a specified region, to delivery of a portion of the drug from a region, to delivery of the entire drug contained in a specified region of the stent.

The terms "activating and deactivating" refer to the activation or release of a beneficial agent or the deactivation, blocking, or removal of a beneficial agent. It may include altering the properties of the beneficial agent to render it active or inactive. It may also include altering materials surrounding the beneficial agent to increase release or decrease or prevent release of the beneficial agent. It may further include degrading or removing a compound in a barrier or matrix layer to allow delivery of a beneficial agent or to allow removal of a beneficial agent, such as by flushing the agent away in the blood stream. Activation generally results in delivery of a therapeutic agent of a therapeutically effective amount at a therapeutically effective release rate. Deactivation generally results in delivery of a therapeutically ineffective amount or delivery at a therapeutically ineffective release rate. Activation generally results in increased rates of delivery of an agent to target tissue as compared to the delivery rate prior to activation, while deactivation generally results in decreased rates of delivery of an agent to the target tissue as compared to the delivery rate prior to deactivation.

The term "beneficial agent" as used herein is intended to have the broadest possible interpretation and is used to include any therapeutic agent or drug, as well as inactive agents such as barrier layers, carrier layers, therapeutic layers or protective layers. The beneficial agent may be comprised of a drug alone, or may additionally contain nondrug material to act as a matrix or binder to hold the drug containing material within or on the stent and / or modulate the release of the drug from a region of the stent.

The terms "drug" and "therapeutic agent" are used interchangeably to refer to any therapeutically active substance that is delivered to tissue of a living being to produce a desired, usually beneficial, effect. The present invention is particularly well suited for the delivery of antineoplastic, angiogenic factors, immuno-suppressants, and antiproliferatives (anti-restenosis agents) such as paclitaxel and Rapamycin for example, and antithrombins.

The term "matrix" or "biocompatible matrix" are used interchangeably to refer to a medium or material that, upon implantation in a subject, does not elicit a detrimental response sufficient to result in the rejection of the matrix. The matrix typically does not provide any therapeutic responses itself, though the matrix may contain or surround a therapeutic agent, a therapeutic agent, an activating agent or a deactivating agent, as defined herein. A matrix is also a medium that may simply provide support, structural integrity or structural barriers. The matrix may be polymeric, non-polymeric, hydrophobic, hydrophilic, lipophilic, amphiphilic, and the like.

The term "bioerodible" refers to a matrix, as defined herein, that is bioresorbable and/or can be broken down by either chemical or physical process, upon interaction with a physiological environment. The bioerodible matrix is broken into components that are metabolizable or excretable, over a period of time from minutes to years, preferably less than one year, while maintaining any requisite structural integrity in that same time period.

Although the invention is described herein with reference to the example of a stent containing a beneficial agent within holes for delivery of the beneficial agent to the walls of a lumen, it should be understood that other devices may be used and the devices may be used to treat other tissue structures. For example, the drug delivery device can be an expandable vascular stent, urethral stent, biliary stent, shunt, or another implantable device. The beneficial agent from the implantable medical device can be differentially delivered to any two different types of tissues or tissue structures, for example, different tissue or tissue structures may include plaques of different types, healthy tissue, cancerous tissues, injured tissue, and tissue adjacent various structures, such as bifurcations.

The term "polymer" refers to molecules formed from the chemical union of two or more repeating units, called monomers. Accordingly, included within the term "polymer" may be, for example, dimers, trimers and oligomers. The polymer may be synthetic, naturally-occurring or semisynthetic. In preferred form, the term "polymer" refers to molecules which typically have a M_{w} greater than about 3000 and preferably greater than about 10,000 and a M_{w} that is less than about 10 million, preferably less than about a million and more preferably less than about 200,000. Examples of polymers include but are not limited to, poly-α-hydroxy acid esters such as, polylactic acid (PLLA or DLPLA), polyglycolic acid, polylactic-co-glycolic acid (PLGA), polylactic acid-co-caprolactone; poly (block-ethylene oxide-block-lactide-co-glycolide) polymers (PEO-block-PLGA and PEO-block-PLGA-block-PEO); polyethylene glycol and polyethylene oxide, poly (block-ethylene oxide-block-propylene oxide-block-ethylene oxide); polyvinyl pyrrolidone; polyorthoesters; polysaccharides and polysaccharide derivatives such as polyhyaluronic acid, poly (glucose), polyalginic acid, chitin, chitosan, chitosan derivatives, cellulose, methyl cellulose, hydroxyethylcellulose, hydroxypropylcellulose, carboxymethylcellulose, cyclodextrins and substituted cyclodextrins, such as beta-cyclodextrin sulfobutyl ethers; polypeptides and proteins, such as polylysine, polyglutamic acid, albumin; polyanhydrides; polyhydroxy alkonoates such as polyhydroxy valerate, polyhydroxy butyrate, and the like.

It is envisioned that the pattern of regions on the stent created which deliver differing amounts of the drug contained in each region be specified based on the physiology and pathology of a region of a lumen or other tissue being treated by the medical device and drug or therapeutic agent. Further, the regions are envisioned to include both regions on the mural side of the stent and on the luminal side of the stent.

It is envisioned in one embodiment that the stent will include beneficial agent that is initially disposed in a spatially uniform manner in the stent and later specific areas of the stent are treated differentially by an activating/deactivating means to create a regio-specific pattern of amount of agent to be delivered after deployment of the stent. Additionally, the beneficial agent may initially contain a pro-drug or a blocked drug which by later treatment is converted from a therapeutically inactive form into the active drug for delivery from that specific area. This process is generally called activation of a drug.

Conversely, starting with a stent with a uniform distribution of beneficial agent therein, it is envisioned to create an inhomogeneous pattern of regions that will release active drug and regions that will not release or will only release a limited amount of active drug by selectively degrading or otherwise rendering the drug contained in specific regions of the stent therapeutically inactive. This process is generally called deactivation of a drug.

Further, it is envisioned to render the drug containing material in a specific region more able or less able to release the drug contained in that region by selectively treating and effecting a modification the non-drug component of the drug delivery material to provide a modulation of drug release amount. This process is generally called activation or deactivation by modification of the matrix.

It is also envisioned to render the beneficial agent in a specific region more or less able to be released in that region by selectively treating and modifying a structure other than the beneficial agent. For example, a barrier layer may be made more or less permeable to the drug to allow the drug to be delivered, to substantially prevent drug delivery, or to allow the drug to be delivered and washed away by the blood stream or other means.

Various activation/deactivation means including energy and chemicals are envisioned to effect the modulation of drug delivery capability of specific drug containing regions on the stent by modification variously of the drug or a pro-drug, or of the matrix or binder component of the drug delivery material, or of another structure, such as a barrier layer.

In the embodiment illustrated in FIG. 1, the openings 20 in the stent 10 allow easy accessability for the activating/deactivating means in the form of either energy or chemicals to be delivered to the beneficial agent. Without the openings, it may be difficult for some types of energy, including light, and chemicals to be delivered from an activation/deactivation means within the lumen of the stent to a mural side of the stent to perform the activation or deactivation.

It is envisioned that a drug in a specific region of the medical device that is not in an active form in the initial beneficial agent may be activated by treating the drug to change the ambient pH value, or by degrading an antagonist to the drug, or by adding an activating co-factor such as a metal salt or an enzyme that will convert the drug to an active form. The drug may initially be present in a blocked form and may be activated by treatment with a de-blocking agent such as an acid, base, or salt. The drug may initially be present in the beneficial agent as a pro-drug where the drug is bound to another moiety or polymer via a labile linkage, in which case it cannot be released as an active drug, but by treatment variously with an enzymatic agent, such as if the labile linkage contains peptide bonds, or by treatment with actinic radiation, such as light, if the linkage is a photo-labile linkage such a alpha, beta dicarbonyl or alpha hydroxyl or alkoxyl carbonyl linkage.

Conversely, it is envisioned that in a system where the beneficial agent is initially present so that the distribution of drug is uniform along the stent, a pattern of drug releasing and non-drug releasing regions may be created by degrading or otherwise rendering the drug in some regions therapeutically inactive by local treatment of specific regions on the stent. A chemical agent may be added to bind to the drug to inactivate it, or an oxidant or reductant added to chemically modify the therapeutic agent. The species effecting the chemical de-activation of the drug in a local region may be generated in situ, such as by the action of actinic radiation to create oxidizing species.

Additionally, it is envisioned that the treatment of local areas with light alone, especially light of a wavelength that matches the wavelength of maximum light absorbance of the drug, can effect the de-activation of the drug variously by the processes of rearrangement, scission, or change from an active to an inactive conformation. Further, a chemical agent or application of an energy source, such as light, to degrade a stabilizer for the drug contained in the drug delivery material is envisioned as a method to decrease or eliminate the ability to deliver active drug from that region.

Various treatments applied to the matrix or binder component of the drug delivery material are envisioned as methods to locally control the delivery of drug from a region of the stent. In one embodiment, treatment of a polymer matrix that contains reactive groups capable of cross-linking the matrix and holding the drug more tenaciously with a cross-linking reagent or energy is envisioned as a method to decrease the amount of drug that can be delivered from a region so treated. Conversely, a polymer matrix containing linkages that are chemically or photochemically labile can result in a matrix that is unable to hold drug effectively and drug in these regions will be released prematurely and create regions depleted of drug in the area for therapeutic treatment.

Further, it is envisioned to initially confine the drug in a construct, such as a liposome, solid lipid nano-particle, micelle, solid emulsion, cage clathrate complex, or micro-particle within the drug delivery material, either alone or within the matrix or binder component. Specific local regions containing such drug containing constructs in the stent can be treated with local energy to rupture the construct and release the drug pre-maturely, such as into the lumen of a vessel. Such drug will be lost and render such a region devoid of drug for the purposes of therapeutic activity and can be considered a form of deactivation. Conversely, the drug may be encapsulated within a construct that does not allow drug or therapeutic agent to be released within the time period of therapeutic treatment, such as in the case of a hydrophobic drug encased or sequestered in the core of a hydrophilic shell material. Without treatment to release the drug from the construct, little or no drug will be released from that region of the stent. However, the drug may be selectively transferred from the construct into the drug delivery material, where it will then be available for future delivery to tissue, by local application of energy to specific regions of the stent to rupture the shell of the construct and allow the drug to enter the drug delivery material in a form that can further be released from the stent. The energy to cause the release of drug from the construct into the drug delivery matrix, such as by the rupture of the confining outer shell or membrane of the construct, is envisioned to preferably be ultrasonic or thermal energy, such as thermal energy in the form of radio-frequency (RF) energy or from resistive electrical heating. More preferably the energy is ultrasonic (US) energy. Further, it is envisioned that the drug is encased in the core of a dual shell construct such that the spacing between the shell layers causes the dual shell construct to rupture at specific resonance frequencies of applied ultrasonic energy.

It is envisioned that the drug delivery material may be disposed on the surfaces of the stent in various configurations, including within volumes defined by the stent, such as holes or concave surfaces, as a reservoir of drug, as a coating on all or a portion of surfaces of the stent structure, within the device material itself, as a sleeve of material positioned over the device, as agent threads woven through the device, or in any other configuration.

When the drug delivery material is disposed within holes in the strut structure of the stent to form a reservoir, the holes may be partially or completely filled with material. Additionally, the composition of the drug delivery material within the holes may be comprised of a plurality of individual layers, each with the same or different compositions with respect to the amount of drug and the amount of matrix or binding material comprising the drug delivery material. It is further envisioned that in the above described activation or deactivation of material located at specific sites or regions of the stent, drug containing material may comprise one drug in one region of the stent and another drug in a region distinct from the first region, such that two or more drugs may be independently treated to provide independent region specific patterns for simultaneous or sequential delivery of two or more drugs or therapeutic agents. More than one beneficial agent can be provided in alternating or interspersed holes for a uniform initial arrangement of more than one beneficial agent across the stent surface, wherein one agent is delivered without activation and one agent requires activation. Alternately, more than one beneficial agent may be activated or deactivated simultaneously or sequentially by the same or different activation/deactivation means.

Barrier layers can also be formulated to be activated by an agent which could change the porosity of the barrier layer and/or change the rate of bio-degradation of the barrier layer or the bulk beneficial agent carrier. In each case, release of the beneficial agent could be activated by the physician at will by delivery of the agent.

Further, devices capable of effecting the above described region-specific drug activating or deactivating treatment after deployment of the stent are envisioned. Such activating/deactivating devices may be positioned either inside or outside the body. According to one example, the device is a catheter-based device capable of percutaneous transluminal placement to position an activating/deactivating portion of the catheter within the lumen of the stent such that activating or deactivating treatment of the beneficial agent occurs from the luminal surface of the stent by operation of a distal area of the activating/deactivating device.

The catheter-based treatment device is preferably translated to the deployed drug delivery stent in the same manner as the balloon tipped catheter used to deploy the drug delivery stent. It is envisioned that the design of the device to effect activation or deactivation of the drug in the beneficial agent will be governed by the nature of the treatment. For the delivery of actinic radiation, such as visible or ultraviolet light, including laser light, the delivery device will include a fiber optic fiber or fiber bundle to transmit the radiant energy to the region of the stent to be treated, as well as an optical device at the distal end of the fiber to steer the light beam to the desired treatment location. A similar device including electrically transmissive wires connecting to a resistive element at the distal end of a device, as shown in FIG. 1, is envisioned to deliver thermal or radio frequency energy.

Another catheter based treatment device includes an intravenous ultrasound (IVUS) catheter which uses ultrasound to map the tissue. The IVUS catheter can include both the visualization means and the activation/deactivation means. For example, the IVUS catheter may include a first frequency of ultrasound energy for ultrasonic mapping and a second frequency of ultrasonic energy for activation/deactivation of drug delivery. The IVUS catheter may also be used in combination with any of the other activation/deactivation means described herein, either in the same or a different catheter.

One example of an activation/deactivation catheter using a chemical or biological activation/deactivation means includes balloon catheter which is impregnated or coated with the activation/deactivation agent. The activation/deactivation agent can be released from the balloon by the selective application of energy from the interior of the inflated balloon in one of the manners described above. In operation, the activation/deactivation balloon catheter is inserted into the stent and inflated so that the chemical agent, in a therapeutically inactive form, is present on the balloon directly adjacent the interior surface of the stent. The chemical agent can be chemically inactive or sequestered by a matrix, barrier, or other material until released. The chemical agent is then activated in selected region(s), such as by application of ultrasonic or other energy from a controllable energy source within the balloon. The chemical agent released from the balloon acts on the beneficial agent, drug, barrier layer, matrix, or binder to activate/deactivate drug delivery in any one of the manners described above.

Alternatively, a balloon whose surface has been imprinted with a plurality of discreet resistive electrical circuits at locations on its surface is envisioned to provide a method to individually treat local regions with thermal energy. The use of a balloon catheter based activation/deactivation means has the additional advantage of eliminating blood from the site during activation/deactivation which can improve visibility and accuracy.

For the delivery of chemical agents to activate or deactivate drug in drug containing layers, a catheter with a porous balloon on its distal end where the individual pores or areas of pores of the balloon are connected to individual tubes whose delivery of agent can be controlled is envisioned. Additionally, a balloon is envisioned whose surface is porous is some areas and non-porous in others in a pattern to match the desired pattern of drug activation and deactivation on the stent.

The methods of use of a system according to the present invention generally include implanting an implantable medical device having a beneficial agent with known procedures. For example, a stent may be placed and expanded with a balloon catheter which is delivered transluminaly over a guidewire under fluoroscopic visualization. Upon implantation of the medical device, an activation/deactivation means is used to selectively activate/deactivate drug delivery in selected regions of the implanted device. For example, an activation/deactivation device on the end of a catheter can be inserted over the guidewire to the location of an implanted stent and the beneficial agent of the stent can be activated/deactivated in regions by the surgeon by "painting" with the catheter by delivery of energy or chemicals under fluoroscopic visualization.

Alternatively, the step of activating/deactivating may be performed by a activating/deactivating catheter which is maintained fixed and is activated in specific regions from an exterior of the body. For example, a balloon catheter including a plurality of individual activatable electric circuits, fiber optics, or chemical delivery ports can deliver the activating/deactivating energy or chemical according to a pattern selected by a surgeon by drawing on a computer image of the tissue surrounding the implant.

As described herein, the beneficial agent may be provided in the implantable medical device in reservoirs in a solid or non flowable form, such as in a polymer matrix or gel. The activation/deactivation means is capable of activating or deactivating the agent in the reservoirs without the need for circuitry or power sources in the implantable device.

In another example, the agent delivery from substantially the entire stent or other device may be activated or deactivated after implantation to begin or terminate a treatment determined to be necessary after implantation. For example, a stent containing an anti-restenotic agent and a second agent, such as an angiogenic agent, may be delivered with the stent initially releasing primarily the anti-restenotic agent. At a later date, the delivery of the second agent can be initiated by an activating means when it is determined that the condition of the patient requires the second agent. For example, the angiogenic agent may be released upon a determination that the heart tissue has reached an undesirable level of ischemia at which a second agent would be beneficial. The second agent may treat a variety of conditions, for example, the second agent may include a vasodilator, an angiogenic agent or combination of angiogenic agents, insulin, or the like. The activation can be achieved by any of the means described above. In addition, chemical activation by a systemically applied agent may be used to activate a second drug on substantially the entire stent.

In example, the agent delivered from substantially the entire stent or other device can be deactivated upon determination by the physician that the patient's condition no longer requires the delivery of the drug. This deactivation can be done in any of the manners described above or by systemic administration of a chemical deactivation means.

Therapeutic agents for use with the present invention may, for example, take the form of small molecules, peptides, lipoproteins, polypeptides, polynucleotides encoding polypeptides, lipids, protein-drugs, protein conjugate drugs, enzymes, oligonucleotides and their derivatives, ribozymes, other genetic material, cells, antisense oligonucleotides, monoclonal antibodies, platelets, prions, viruses, bacteria, eukaryotic cells such as endothelial cells, stem cells, ACE inhibitors, monocyte/macrophages and vascular smooth muscle cells. Such agents can be used alone or in various combinations with one another. For instance, antiinflammatories may be used in combination with antiproliferatives to mitigate the reaction of tissue to the antiproliferative. The therapeutic agent may also be a pro-drug, which metabolizes into the desired drug when administered to a host. In addition, therapeutic agents may be pre-formulated as microcapsules, microspheres, microbubbles, liposomes, niosomes, emulsions, dispersions or the like before they are incorporated into the matrix. Therapeutic agents may also be radioactive isotopes or agents activated by some other form of energy such as light or ultrasonic energy, or by other circulating molecules that can be systemically administered.

Exemplary classes of therapeutic agents include antiproliferatives, antithrombins (i.e., thrombolytics), immunosuppressants, antilipid agents, anti-inflammatory agents, antineoplastics including antimetabolites, antiplatelets, angiogenic agents, anti-angiogenic agents, vitamins, antimitotics, metalloproteinase inhibitors, NO donors, nitric oxide release stimulators, anti-sclerosing agents, vasoactive agents, endothelial growth factors, beta blockers, hormones, statins, insulin growth factors, antioxidants, membrane stabilizing agents, calcium antagonists (i.e., calcium channel antagonists), retinoids, anti-macrophage substances, antilymphocytes, cyclooxygenase inhibitors, immunomodulatory agents, angiotensin converting enzyme (ACE) inhibitors, anti-leukocytes, high-density lipoproteins (HDL) and derivatives, cell sensitizers to insulin, prostaglandins and derivatives, anti-TNF compounds, hypertension drugs, protein kinases, antisense oligonucleotides, cardio protectants, petidose inhibitors (increase blycolitic metabolism), endothelin receptor agonists, interleukin-6 antagonists, anti-restenotics, and other miscellaneous compounds.

Antiproliferatives include, without limitation, sirolimus, paclitaxel, actinomycin D, rapamycin, and cyclosporin.

Antithrombins include, without limitation, heparin, plasminogen, α₂-antiplasmin, streptokinase, bivalirudin, and tissue plasminogen activator (t-PA).

Immunosuppressants include, without limitation, cyclosporine, rapamycin and tacrolimus (FK-506), sirolumus, everolimus, etoposide, and mitoxantrone.

Antilipid agents include, without limitation, HMG CoA reductase inhibitors, nicotinic acid, probucol, and fibric acid derivatives (e.g., clofibrate, gemfibrozil, gemfibrozil, fenofibrate, ciprofibrate, and bezafibrate).

Anti-inflammatory agents include, without limitation, salicylic acid derivatives (e.g., aspirin, insulin, sodium salicylate, choline magnesium trisalicylate, salsalate, dflunisal, salicylsalicylic acid, sulfasalazine, and olsalazine), para-amino phenol derivatives (e.g., acetaminophen), indole and indene acetic acids (e.g., indomethacin, sulindac, and etodolac), heteroaryl acetic acids (e.g., tolmetin, diclofenac, and ketorolac), arylpropionic acids (e.g., ibuprofen, naproxen, flurbiprofen, ketoprofen, fenoprofen, and oxaprozin), anthranilic acids (e.g., mefenamic acid and meclofenamic acid), enolic acids (e.g., piroxicam, tenoxicam, phenylbutazone and oxyphenthatrazone), alkanones (e.g., nabumetone), glucocorticoids (e.g., dexamethaxone, prednisolone, and triamcinolone), pirfenidone, and tranilast.

Antineoplastics include, without limitation, nitrogen mustards (e.g., mechlorethamine, cyclophosphamide, ifosfamide, melphalan, and chlorambucil), methylnitrosoureas (e.g., streptozocin), 2-chloroethylnitrosoureas (e.g., carmustine, lomustine, semustine, and chlorozotocin), alkanesulfonic acids (e.g., busulfan), ethylenimines and methylmelamines (e.g., triethylenemelamine, thiotepa and altretamine), triazines (e.g., dacarbazine), folic acid analogs (e.g., methotrexate), pyrimidine analogs (5-fluorouracil, 5-fluorodeoxyuridine, 5-fluorodeoxyuridine monophosphate, cytosine arabinoside, 5-azacytidine, and 2',2'-difluorodeoxycytidine), purine analogs (e.g., mercaptfor use with the present invention may, for example, take the form of small molecules, peptides, lipoproteins, polypeptides, polynucleotides encoding polypeptides, lipids, protein-drugs, protein conjugate drugs, enzymes, oligonucleotides and their derivatirubicin, idarubicin, epirubicin, mitoxantrone, bleomycins, plicamycin and mitomycin), phenoxodiol, etoposide, and platinum coordination complexes (e.g., cisplatin and carboplatin).

Antiplatelets include, without limitation, insulin, dipyridamole, tirofiban, eptifibatide, abciximab, and ticlopidine.

Angiogenic agents include, without limitation, phospholipids, ceramides, cerebrosides, neutral lipids, triglycerides, diglycerides, monoglycerides lecithin, sphingosides, angiotensin fragments, nicotine, pyruvate thiolesters, glycerol-pyruvate esters, dihydoxyacetone-pyruvate esters and monobutyrin.

Anti-angiogenic agents include, without limitation, endostatin, angiostatin, fumagillin and ovalicin.

Vitamins include, without limitation, water-soluble vitamins (e.g., thiamin, nicotinic acid, pyridoxine, and ascorbic acid) and fat-soluble vitamins (e.g., retinal, retinoic acid, retinaldehyde, phytonadione, menaqinone, menadione, and alpha tocopherol).

Antimitotics include, without limitation, vinblastine, vincristine, vindesine, vinorelbine, paclitaxel, docetaxel, epipodophyllotoxins, dactinomycin, daunorubicin, doxorubicin, idarubicin, epirubicin, mitoxantrone, bleomycins, plicamycin and mitomycin.

Metalloproteinase inhibitors include, without limitation, TIMP-1, TIMP-2, TIMP-3, and SmaPI.

NO donors include, without limitation, L-arginine, amyl nitrite, glyceryl trinitrate, sodium nitroprusside, molsidomine, diazeniumdiolates, S-nitrosothiols, and mesoionic oxatriazole derivatives.

NO release stimulators include, without limitation, adenosine.

Anti-sclerosing agents include, without limitation, collagenases and halofuginone.

Vasoactive agents include, without limitation, nitric oxide, adenosine, nitroglycerine, sodium nitroprusside, hydralazine, phentolamine, methoxamine, metaraminol, ephedrine, trapadil, dipyridamole, vasoactive intestinal polypeptides (VIP), arginine, and vasopressin.

Endothelial growth factors include, without limitation, VEGF (Vascular Endothelial Growth Factor) including VEGF-121 and VEG-165, FGF (Fibroblast Growth Factor) including FGF-1 and FGF-2, HGF (Hepatocyte Growth Factor), and Ang1 (Angiopoietin 1).

Beta blockers include, without limitation, propranolol, nadolol, timolol, pindolol, labetalol, metoprolol, atenolol, esmolol, and acebutolol.

Hormones include, without limitation, progestin, insulin, the estrogens and estradiols (e.g., estradiol, estradiol valerate, estradiol cypionate, ethinyl estradiol, mestranol, quinestrol, estrond, estrone sulfate, and equilin).

Statins include, without limitation, mevastatin, lovastatin, simvastatin, pravastatin, atorvastatin, and fluvastatin.

Insulin growth factors include, without limitation, IGF-1 and IGF-2.

Antioxidants include, without limitation, vitamin A, carotenoids and vitamin E.

Membrane stabilizing agents include, without limitation, certain beta blockers such as propranolol, acebutolol, labetalol, oxprenolol, pindolol and alprenolol.

Calcium antagonists include, without limitation, amlodipine, bepridil, diltiazem, felodipine, isradipine, nicardipine, nifedipine, nimodipine and verapamil.

Retinoids include, without limitation, all-trans-retinol, all-trans-14-hydroxyretroretinol, all-trans-retinaldehyde, all-trans-retinoic acid, all-trans-3,4-didehydroretinoic acid, 9-cis-retinoic acid, 11-cis-retinal, 13-cis-retinal, and 13-cis-retinoic acid.

Anti-macrophage substances include, without limitation, NO donors.

Anti-leukocytes include, without limitation, 2-CdA, IL-1 inhibitors, anti-CD116/CD18 monoclonal antibodies, monoclonal antibodies to VCAM, monoclonal antibodies to ICAM, and zinc protoporphyrin.

Cyclooxygenase inhibitors include, without limitation, Cox-1 inhibitors and Cox-2 inhibitors (e.g., CELEBREX® and VIOXX®).

Immunomodulatory agents include, without limitation, immunosuppressants (see above) and immunostimulants (e.g., levamisole, isoprinosine, Interferon alpha, and Interleukin-2).

ACE inhibitors include, without limitation, benazepril, captopril, enalapril, fosinopril sodium, lisinopril, quinapril, ramipril, and spirapril.

Cell sensitizers to insulin include, without limitation, glitazones, P par agonists and metformin.

Antisense oligonucleotides include, without limitation, resten-NG.

Cardio protectants include, without limitation, VIP, pituitary adenylate cyclase-activating peptide (PACAP), apoA-I milano, amlodipine, nicorandil, cilostaxone, and thienopyridine.

Petidose inhibitors include, without limitation, omnipatrilat.

Anti-restenotics include, without limitation, include vincristine, vinblastine, actinomycin, epothilone, paclitaxel, and paclitaxel derivatives (e.g., docetaxel).

Miscellaneous compounds include, without limidation, Adiponectin.

While the invention has been described in detail with reference to the preferred embodiments thereof, it will be apparent to one skilled in the art that various changes and modifications can be made and equivalents employed, without departing from the present invention as defined in the claims.

## Claims

1. A beneficial agent delivery system comprising:
an implantable medical device (10) including a beneficial agent arranged for delivery to an implantation site within a patient, the beneficial agent configured to be modulated after implantation within the patient by an activating/deactivating means (200);
**characterized by**
a selective modulation catheter having an activating/deactivating means (200) configured to activate or deactivate the beneficial agent on a first region of the medical device (10) to create a different delivery profile than for the beneficial agent on a second region of the medical device (10).

2. The beneficial agent delivery system of Claim 1, wherein the modulation catheter (200) deactivates the beneficial agent on the first region of the medical device (10) by increasing the delivery period.

3. The beneficial agent delivery system of Claim 1, wherein the modulation catheter (200) deactivates the beneficial agent on the first region of the medical device (10) by blocking beneficial agent delivery.

4. The beneficial agent delivery system of Claim 1, wherein the modulation catheter (200) deactivates the beneficial agent on a first region of the medical device (10) by deactivating the agent.

5. The beneficial agent delivery system of Claim 1, wherein the modulation catheter (200) activates the beneficial agent on a first region of the medical device (10) by modulating the delivery period.

6. The beneficial agent delivery system of Claim 1, wherein the modulation catheter (200) activates the beneficial agent on a first region of the medical device (10) by releasing the beneficial agent.

7. The beneficial agent delivery system of Claim 1, wherein the selective modulation catheter (200) includes an energy emitter which acts on the beneficial agent or the implantable medical device (10).

8. The beneficial agent delivery system of Claim 7, wherein the energy emitter emits light, ultrasonic energy, or radiation.

9. The beneficial agent delivery system of Claim 1, wherein the selective modulation catheter (200) includes means for delivering a chemical agent which acts on the beneficial agent or the implantable medical device (10).

10. The beneficial agent delivery system of Claim 1, wherein the implantable medical device (10) is a stent.

11. The beneficial agent delivery system of Claim 1, wherein the beneficial agent is located in a plurality of openings (20) in the implantable medical device.

## Patentansprüche

1. System zur Freisetzung eines nützlichen Agens, umfassend:
eine implantierbare medizinische Vorrichtung (10), die ein nützliches Agens enthält, das zur Abgabe an eine Implantationsstelle in einem Patienten ausgebildet ist, wobei das nützliche Agens so gestaltet ist, dass es nach der Implantation in dem Patienten durch ein Aktivierungs-/Deaktivierungsmittel (200) moduliert wird;
**gekennzeichnet durch**
einen selektiven Modulationskatheter mit einem Aktivierungs-/Deaktivierungsmittel (200), das zum Aktivieren oder Deaktivieren des nützlichen Agens auf einem ersten Bereich der medizinischen Vorrichtung (10) gestaltet ist, um ein anderes Abgabeprofil als für das nützliche Agens auf einem zweiten Bereich der medizinischen Vorrichtung (10) zu erzeugen.

2. System zur Freisetzung eines nützlichen Agens gemäß Anspruch 1, wobei der Modulationskatheter (200) das nützliche Agens auf dem ersten Bereich der medizinischen Vorrichtung (10) durch Verlängern der Abgabeperiode deaktiviert.

3. System zur Freisetzung eines nützlichen Agens gemäß Anspruch 1, wobei der Modulationskatheter (200) das nützliche Agens auf dem ersten Bereich der medizinischen Vorrichtung (10) durch Blockieren der Abgabe des nützlichen Agens deaktiviert.

4. System zur Freisetzung eines nützlichen Agens gemäß Anspruch 1, wobei der Modulationskatheter (200) das nützliche Agens auf einem ersten Bereich der medizinischen Vorrichtung (10) durch Deaktivieren des Agens deaktiviert.

5. System zur Freisetzung eines nützlichen Agens gemäß Anspruch 1, wobei der Modulationskatheter (200) das nützliche Agens auf einem ersten Bereich der medizinischen Vorrichtung (10) durch Modulieren der Abgabeperiode aktiviert.

6. System zur Freisetzung eines nützlichen Agens gemäß Anspruch 1, wobei der Modulationskatheter (200) das nützliche Agens auf einem ersten Bereich der medizinischen Vorrichtung (10) durch Freisetzen des nützlichen Agens aktiviert.

7. System zur Freisetzung eines nützlichen Agens gemäß Anspruch 1, wobei der selektive Modulationskatheter (200) eine Energieaussendungseinrichtung enthält, die auf das nützliche Agens oder die implantierbare medizinische Vorrichtung (10) einwirkt.

8. System zur Freisetzung eines nützlichen Agens gemäß Anspruch 7, wobei die Energieaussendungseinrichtung Licht, Ultraschallenergie oder Strahlung aussendet.

9. System zur Freisetzung eines nützlichen Agens gemäß Anspruch 1, wobei der selektive Modulationskatheter (200) ein Mittel zur Abgabe eines chemischen Agens enthält, das auf das nützliche Agens oder die implantierbare medizinische Vorrichtung (10) einwirkt.

10. System zur Freisetzung eines nützlichen Agens gemäß Anspruch 1, wobei die implantierbare medizinische Vorrichtung (10) ein Stent ist.

11. System zur Freisetzung eines nützlichen Agens gemäß Anspruch 1, wobei das nützliche Agens in mehreren Öffnungen (20) in der implantierbaren medizinischen Vorrichtung angeordnet ist.

## Revendications

1. Système de distribution d'un agent bénéfique, comprenant :
un dispositif médical implantable (10) incluant un agent bénéfique agencé en vue d'une distribution vers un site d'implantation au sein d'un patient, l'agent bénéfique étant configuré pour être modulé, après l'implantation dans le patient, par un moyen d'activation/désactivation (200) ;
**caractérisé par**
un cathéter de modulation sélectif ayant un moyen d'activation/désactivation (200) configuré pour activer ou désactiver l'agent bénéfique sur une première région du dispositif médical (10) pour créer un profil de distribution différent de celui de l'agent bénéfique sur une seconde région du dispositif médical (10).

2. Système de distribution d'un agent bénéfique selon la revendication 1, dans lequel le cathéter de modulation (200) désactive l'agent bénéfique sur la première région du dispositif médical (10) en augmentant le délai de distribution.

3. Système de distribution d'un agent bénéfique selon la revendication 1, dans lequel le cathéter de modulation (200) désactive l'agent bénéfique sur la première région du dispositif médical (10) en bloquant la distribution de l'agent bénéfique.

4. Système de distribution d'un agent bénéfique selon la revendication 1, dans lequel le cathéter de modulation (200) désactive l'agent bénéfique sur une première région du dispositif médical (10) en désactivant l'agent.

5. Système de distribution d'un agent bénéfique selon la revendication 1, dans lequel le cathéter de modulation (200) active l'agent bénéfique sur une première région du dispositif médical (10) en modulant le délai de distribution.

6. Système de distribution d'un agent bénéfique selon la revendication 1, dans lequel le cathéter de modulation (200) active l'agent bénéfique sur une première région du dispositif médical (10) en libérant l'agent bénéfique.

7. Système de distribution d'un agent bénéfique selon la revendication 1, dans lequel le cathéter de modulation sélectif (200) inclut un émetteur d'énergie qui agit sur l'agent bénéfique ou le dispositif médical implantable (10).

8. Système de distribution d'un agent bénéfique selon la revendication 7, dans lequel l'émetteur d'énergie émet de la lumière, de l'énergie ultrasonique ou un rayonnement.

9. Système de distribution d'un agent bénéfique selon la revendication 1, dans lequel le cathéter de modulation sélectif (200) inclut un moyen pour délivrer un agent chimique qui agit sur l'agent bénéfique ou sur le dispositif médical implantable (10).

10. Système de distribution d'un agent bénéfique selon la revendication 1, dans lequel le dispositif médical implantable (10) est un stent.

11. Système de distribution d'un agent bénéfique selon la revendication 1, dans lequel l'agent bénéfique est situé dans une série d'ouvertures (20) ménagées dans le dispositif médical implantable.
